# FASCICULE DE BREVET EUROPEEN

(11) **EP 3 554 602 B1**
(45) Date de publication et mention de la délivrance du brevet: **21.10.2020**
(21) Numéro de dépôt: 17828946.8
(22) Date de dépôt: 14.12.2017
(51) Int. Cl.: A61M 15/08

(54) **ENSEMBLE DE DISTRIBUTION NASALE DE PRODUIT FLUIDE**
VORRICHTUNG ZUR NASALEN ABGABE EINES FLUIDPRODUKTS
ASSEMBLY FOR NASAL DISPENSING OF FLUID PRODUCT

(30) Priorité: 15.12.2016 FR 1662561
(43) Date de publication de la demande: 23.10.2019
(73) Titulaire: APTAR France SAS, 27110 Le Neubourg (FR)
(72) Inventeur: GRAINE, Lila, 78650 Beynes (FR); PIAZZONI, Eric, 27370 Saint Cyr la Campagne (FR); BOIVIN, Pierre, 27430 Saint Etienne du Vauvray (FR)
(74) Mandataire: CAPRI
(86) Numéro de dépôt international: PCT/FR2017/053588
(87) Numéro de publication internationale: WO 2018/109409

(56) Documents cités:
- DE-U1-202015 006 433
- US-A- 1 155 608
- US-A1- 2011 132 354

## Description

La présente invention concerne un ensemble de distribution comportant un dispositif de distribution nasale de produit fluide.

Les dispositifs de distribution nasale sont bien connus. Ils comportent généralement un réservoir contenant une ou plusieurs doses de produits fluide et une tête de distribution mobile par rapport audit réservoir pour distribuer le produit fluide, notamment via une pompe, une valve doseuse ou un piston coulissant dans ledit réservoir. Lorsque l'utilisateur souhaite utiliser le dispositif, il insère la tête de distribution dans la narine et actionne le dispositif pour distribuer une dose de produit fluide, généralement sous forme de spray.

Un inconvénient avec les dispositifs de l'art antérieur concerne l'efficacité de la dose distribuée dans la narine, en particulier lorsque le produit fluide distribué a pour objectif d'agir sur le cerveau. En effet, seule une partie minime de la dose atteint généralement la zone cible pour ce type de traitement, à savoir la zone olfactive comprenant les éthmoïdes, notamment en raison d'une orientation du dispositif d'administration dans la narine qui est variable d'un patient à un autre. Or, il apparait que cette orientation conditionne la bonne visée de la zone cible, en particulier dans le cas d'un spray fermé utilisé pour obtenir le maximum de déposition dans la zone cible.

Le document US1155608 décrit un dispositif d'inhalation nasale.

La présente invention a pour but de fournir un ensemble de distribution nasale qui ne reproduit pas les inconvénients susmentionnés.

La présente invention a notamment pour but de fournir un ensemble de distribution nasale permettant de maîtriser l'orientation du dispositif dans la narine, quelle que soit la morphologie du patient.

La présente invention a aussi pour but de fournir un ensemble de distribution nasale qui améliore le taux de déposition de produit actif sur la zone olfactive et/ou les éthmoïdes.

La présente invention a également pour but de fournir un ensemble de distribution nasale qui est simple et peu coûteux à fabriquer et à assembler.

La présente a donc pour objet un ensemble de distribution comportant un dispositif de distribution nasale de produit fluide comportant un corps formant réservoir contenant du produit fluide ou supportant fixement un tel réservoir, une tête de distribution étant assemblée sur ledit corps, ladite tête de distribution étant pourvue d'un orifice de distribution, ledit ensemble comportant un organe de positionnement qui coopère avec ledit dispositif de distribution nasale, ledit organe de positionnement comportant au moins une première zone d'appui facial, qui lors de l'actionnement, coopère avec le front ou la base du nez de l'utilisateur, ladite tête de distribution étant axialement déplaçable par rapport audit corps, ledit organe de positionnement comportant au moins un manchon creux dans lequel est insérée de manière serrante ladite tête de distribution, ledit organe de positionnement comportant une seconde zone d'appui facial adaptée à coopérer avec la lèvre supérieure de l'utilisateur.

Avantageusement, ledit organe de positionnement comporte deux manchons creux, chacun adapté à une narine respective.

Avantageusement, ledit organe de positionnement comporte des troisièmes zones d'appui, avantageusement amovibles, adaptées à coopérer avec les côtés du visage et/ou avec les oreilles de l'utilisateur.

Avantageusement, ledit organe de positionnement comporte une structure de support formant une surface d'appui proximale adaptée à recevoir, lors de l'actionnement, au moins un doigt, avantageusement deux doigts, de l'utilisateur.

Avantageusement, ladite structure de support forme ledit au moins un manchon creux et ladite seconde zone d'appui facial.

Avantageusement, ledit organe de positionnement comporte un élément de liaison reliant ladite structure de support à ladite première zone d'appui facial.

Avantageusement, ledit élément de liaison est réglable en trois dimensions par rapport à ladite structure de support.

Avantageusement, l'angle d'insertion vertical de la tête de distribution dans la narine est compris entre 30° et 60°, avantageusement entre 40° et 50°, de préférence environ 45°.

Avantageusement, l'angle d'insertion horizontal de la tête de distribution dans la narine est compris entre 0° et 10°, avantageusement environ 5°.

Avantageusement, la profondeur d'insertion de la tête de distribution dans la narine est comprise entre 5 mm et 15 mm, avantageusement environ 10 mm.

Selon une première variante avantageuse, ledit réservoir contient une seule ou seulement deux dose(s) de produit fluide.

Selon une seconde variante avantageuse, ledit réservoir contient une pluralité de doses de produit fluide.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective de côté d'un dispositif de distribution nasale selon un premier mode de réalisation avantageux de l'invention, en position d'utilisation,
- la figure 2 est une vue schématique en perspective de face du dispositif de la figure 1, en position d'utilisation,
- la figure 3 est une vue schématique en perspective illustrant une manière d'utiliser le dispositif des figures 1 et 2,
- la figure 4 est une vue similaire à celle de la figure 1 d'un dispositif de distribution nasale selon un second mode de réalisation avantageux de l'invention, en position d'utilisation,
- la figure 5 est une vue schématique en perspective de face du dispositif de la figure 4, en position d'utilisation,
- les figures 6 à 9 sont des vues de détail de divers réglages de l'élément de liaison par rapport à la structure de support, et
- les figures 10 et 11 sont des graphiques illustrant des tests comparatifs de déposition dans le nez, à différents angles d'insertion, respectivement à deux profondeurs d'insertion différentes dans la narine.

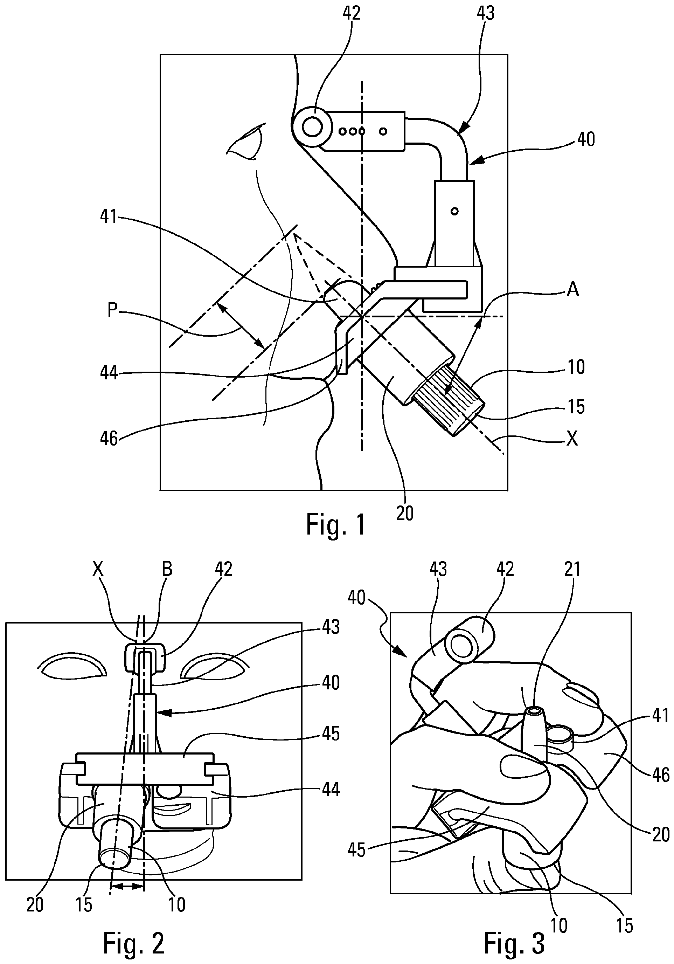

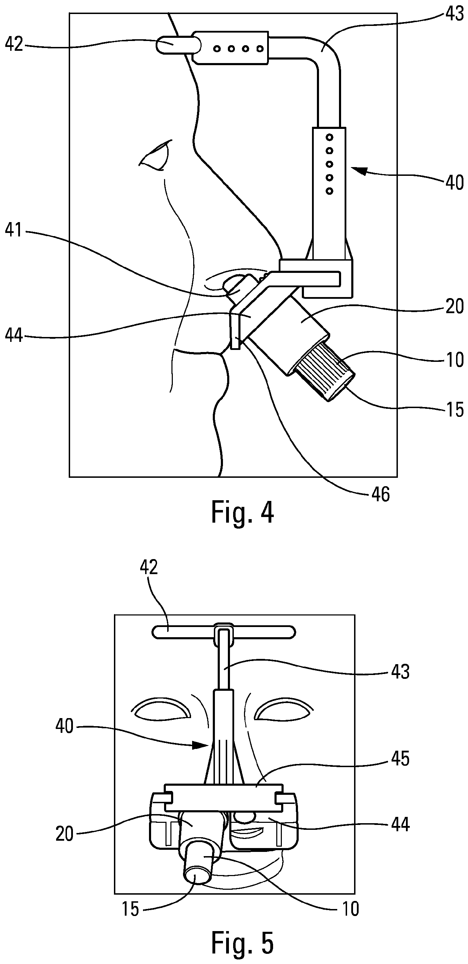

Dans la description, les termes "axial" et "radial" se réfèrent à l'axe longitudinal X du dispositif représenté sur les figures 1 et 4. Les termes "proximal" et "distal" se réfèrent à l'orifice de distribution. Les termes "horizontal" et "vertical" se réfèrent à la position droite d'un utilisateur représentée sur les figures 1 et 4.

Le dispositif de distribution de produit fluide représenté sur les figures 1 à 5 comporte classiquement un corps 10 supportant fixement un réservoir (non représenté) contenant du produit fluide. Une tête de distribution 20 est assemblée sur ledit corps 10 de manière axialement déplaçable par rapport audit corps 10, ladite tête de distribution 20 étant pourvue d'un orifice de distribution 21. En variante, le corps 10 peut former directement le réservoir, auquel cas la tête de distribution 20 serait montée directement sur le réservoir.

De manière connue, ladite tête de distribution 20 comporte une surface d'appui proximale pouvant recevoir lors de l'actionnement au moins un doigt, typiquement deux doigts, de l'utilisateur. De même, le corps 10 comporte une surface d'appui distale 15 pouvant recevoir lors de l'actionnement un doigt, typiquement le pouce, de l'utilisateur. D'autres moyens d'actionnement, par exemple du type actionnement latéral sont toutefois aussi envisageables.

Lorsque le dispositif est multidoses, une pompe ou une valve doseuse (non représentée) est montée sur le corps, pour distribuer une dose de produit fluide à chaque actionnement.

Lorsque le dispositif est un unidose (le réservoir ne contient qu'une seule dose) ou un bidose (le réservoir contient deux doses, généralement une pour chaque narine), un piston (non représenté) est monté coulissant dans le réservoir, ledit piston étant, lors de l'actionnement, déplacé par la tête de distribution 20 lorsque celle-ci se déplace axialement par rapport au corps 10.

Selon l'invention, le dispositif comporte un organe de positionnement 40 qui coopère, de préférence de manière amovible, avec ladite tête de distribution 20.

Ledit organe de positionnement 40 comporte au moins un manchon creux 41 dans lequel est insérée de manière serrante ladite tête de distribution 20.

De préférence, ledit organe de positionnement 40 comporte deux manchons creux 41, chacun adapté à une narine respective, comme visible sur les figures 2 et 5.

Cet organe de positionnement 40 comporte au moins une première zone d'appui facial qui, lors de l'actionnement, coopère avec le visage de l'utilisateur.

Cette première zone d'appui facial 42 est adaptée à coopérer avec le front ou la base du nez. Comme visible sur les figures, cette première zone d'appui facial 42 est avantageusement de forme arrondie.

Dans l'exemple des figures 1 à 3, la première zone d'appui facial 42 est réalisée sous la forme d'un cylindre s'appuyant sur la base du nez, entre les deux yeux.

Dans l'exemple des figures 4 et 5, la première zone d'appui facial 42 est réalisée sous la forme d'une tige horizontale s'appuyant sur le front de l'utilisateur, et s'étendant latéralement de part et d'autre du nez. En variante, on pourrait envisager deux ou plusieurs zones d'appui décalées latéralement de part et d'autre du nez, pour améliorer la stabilité de l'ensemble en utilisation.

Bien entendu, toute autre forme appropriée est envisageable pour la première zone d'appui facial 42.

La première zone d'appui facial 42 est solidaire d'un élément de liaison 43, qui sera plus amplement décrit ci-après.

Une seconde zone d'appui facial 46, adaptée à coopérer avec la lèvre supérieure, est également prévue.

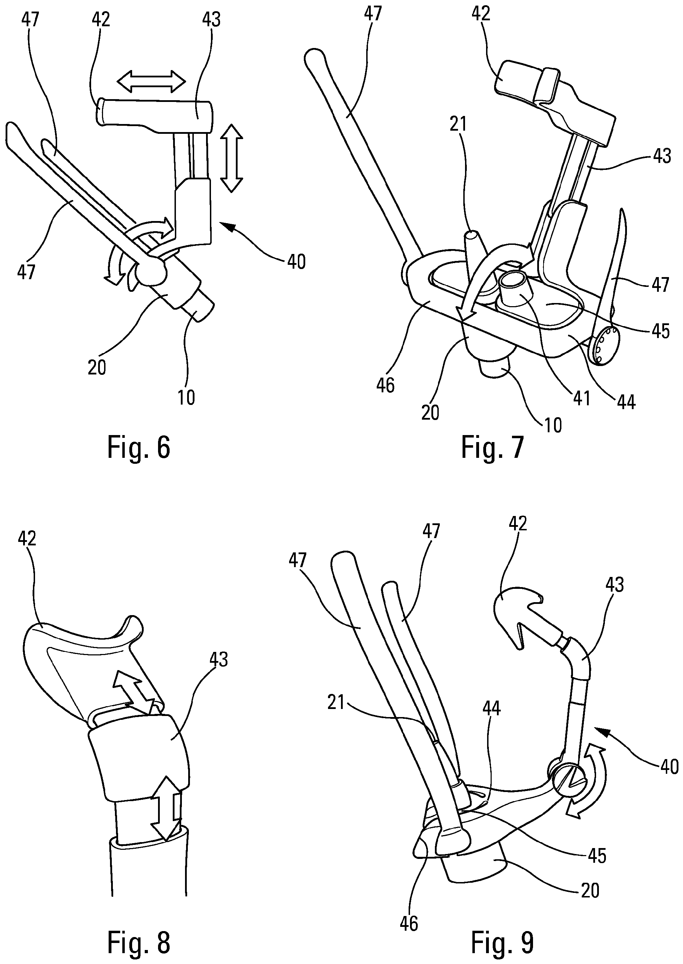

Avantageusement, des troisièmes zones d'appui 47, par exemple sous forme de banches ou de tiges adaptées à coopérer avec les côtés du visage et/ou avec les oreilles de l'utilisateur, peuvent être prévues. Ces branches 47 sont visibles sur les figures 6, 7 et 9. De telles branches 47 peuvent servir à régler la bonne position de l'organe de positionnement 40, et peuvent donc être amovibles après réglage pour ne pas gêner l'actionnement du dispositif en utilisation.

Avantageusement, l'organe de positionnement 40 comporte une structure de support 44 formant une surface d'appui proximale 45 adaptée à recevoir, lors de l'actionnement, au moins un doigt, avantageusement deux doigts, de l'utilisateur, comme visible sur la figure 3. Cette surface d'appui proximale 45 recouvre donc la surface d'appui correspondante de la tête de distribution 20, de sorte que le mouvement d'actionnement du dispositif reste le même, malgré la présence de l'organe de positionnement 40. En variante, le dispositif peut aussi être actionné avec les deux mains positionnées sur la surface d'appui proximale 45, de part et d'autre de la tête de distribution 20.

De manière avantageuse, cette structure de support 44 intègre également le ou les manchon(s) creux 41, ainsi que la seconde zone d'appui facial 46.

La seconde zone d'appui facial 46 est avantageusement réalisée par une surface inclinée par rapport à ladite surface d'appui proximale 45.

D'autres formes appropriées sont possibles pour cette seconde zone d'appui facial 46.

L'élément de liaison 43 permet de reliant ladite structure de support 44 à ladite première zone d'appui facial 42. Il peut être réglable en trois dimensions sur ladite structure de support 44, par exemple au moyen de parties coulissantes et/ou pivotantes, comme illustré schématiquement à titre d'exemple sur les figures 6 à 9. Ceci permet d'ajuster avec précision le positionnement de la structure de support 44, et donc de la tête de distribution 20, par rapport à l'élément de liaison 43 et donc la première zone d'appui facial 42. De préférence, le positionnement angulaire horizontal de la tête de distribution 20 dans la narine, c'est-à-dire l'angle entre les axes X et B sur la figure 2, est fixe, pour garantir la bonne orientation du dispositif lors de l'actionnement.

De manière similaire, on peut envisager une première zone d'appui facial réglable, notamment en hauteur et en largeur, pour s'adapter à la morphologie de l'utilisateur

Dans les exemples, l'élément de liaison 43 est formé par une tige courbée, mais d'autres formes appropriées sont envisageables.

L'organe de positionnement 40 permet d'orienter la tête de distribution 20 dans la narine d'une manière sensiblement prédéterminée.

Ainsi, l'angle d'insertion vertical de la tête de distribution 20 dans la narine, c'est-à-dire l'angle entre les axes X et A sur la figure 1, est compris entre 30° et 60°, avantageusement entre 40° et 50°, de préférence environ 45°.

De même, l'angle d'insertion horizontal de la tête de distribution 20 dans la narine, c'est-à-dire l'angle entre les axes X et B sur la figure 2, est compris entre 0° et 10°, avantageusement environ 5°.

Par ailleurs, la profondeur d'insertion P de la tête de distribution 20 dans la narine est comprise entre 5 mm et 15 mm, avantageusement environ 10 mm.

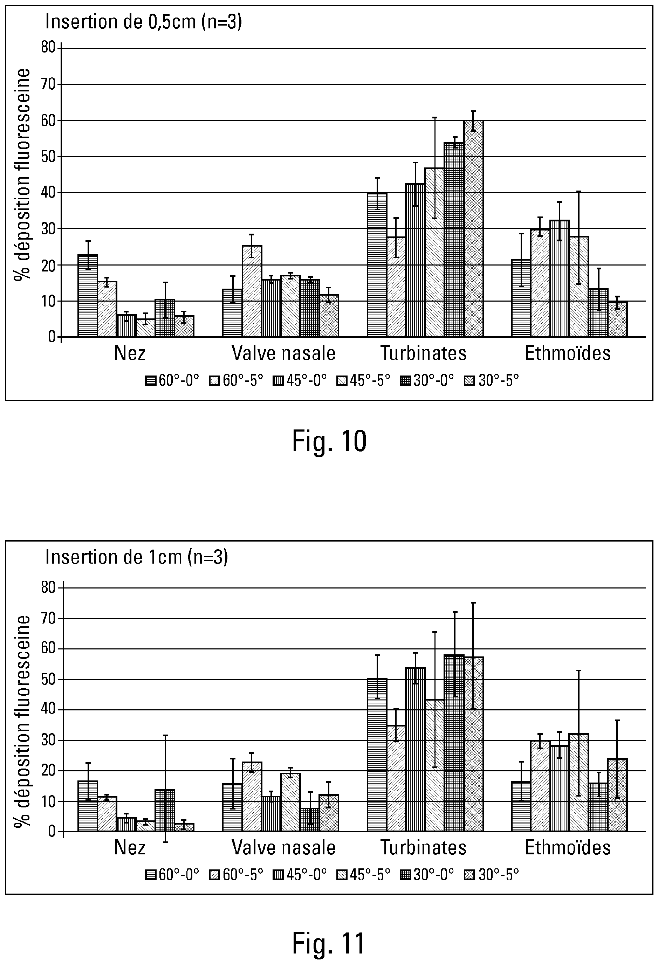

Les figures 10 et 11 illustrent des tests comparatifs de déposition du produit dans les différentes parties du nez : l'entrée, ici appelé nez, la valve nasale, les turbinates et les éthmoïdes, qui sont la cible privilégiée.

La figure 10 montre les résultats à différents angles verticaux (tests réalisés à 30°, 45° et 60°) et horizontaux (tests réalisés à 0° et 5°) pour une profondeur d'insertion P de 5 mm, et la figure 11 pour une profondeur d'insertion P de 10 mm.

On constate que les meilleurs résultats au niveau des éthmoïdes sont réalisés avec un angle vertical de 45°. Lorsque la profondeur d'insertion P est de 5 mm, un angle horizontal de 0° associé à l'angle vertical de 45° est optimal. Lorsque la profondeur d'insertion P est de 10 mm, un angle horizontal de 5° associé à l'angle vertical de 45° est optimal.

On constate aussi qu'à 45° d'angle vertical, la différence entre un angle horizontal de 0° et un angle horizontal de 5° existe, mais n'est pas importante, et ce quelle que soit la profondeur d'insertion. Au contraire, à 60° d'angle vertical, la différence devient importante.

Il s'en déduit que la combinaison idéale correspond à une profondeur d'insertion P d'environ 10 mm, un angle vertical d'environ 45° et un angle horizontal d'environ 5°.

La présente invention est particulièrement adaptée dans le cas de traitements de maladies neuro dégénératives, type Parkinson ou Alzheimer, nécessitant de faire transiter le médicament au travers de la barrière nez-cerveau. L'atteinte de la zone cible, en particulier les ethmoïdes, par la cavité nasale est un moyen simplifié et non invasif d'administrer un traitement dans le cerveau.

La présente invention a été décrite en référence à diverses variantes de réalisation avantageuses, mais il est entendu qu'un homme du métier peut y apporter toutes modifications, sans sortir du cadre de la présente invention tel que défini par les revendications annexées.

## Revendications

1. Ensemble de distribution comportant un dispositif de distribution nasale de produit fluide comportant un corps (10) formant réservoir contenant du produit fluide ou supportant fixement un tel réservoir, une tête de distribution (20) étant assemblée sur ledit corps (10), ladite tête de distribution (20) étant pourvue d'un orifice de distribution (21), ledit ensemble comportant un organe de positionnement (40) qui coopère avec ledit dispositif de distribution nasale, ledit organe de positionnement (40) comportant au moins une première zone d'appui facial (42), qui lors de l'actionnement, coopère avec le front ou la base du nez de l'utilisateur, **caractérisé en ce que** ladite tête de distribution (20) est axialement déplaçable par rapport audit corps (10), ledit organe de positionnement (40) comportant au moins un manchon creux (41) dans lequel est insérée de manière serrante ladite tête de distribution (20), ledit organe de positionnement (40) comportant une seconde zone d'appui facial (46) adaptée à coopérer avec la lèvre supérieure de l'utilisateur.

2. Ensemble selon la revendication 1, dans lequel ledit organe de positionnement (40) comporte deux manchons creux (41), chacun adapté à une narine respective.

3. Ensemble selon l'une quelconque des revendications précédentes, dans lequel ledit organe de positionnement (40) comporte des troisièmes zones d'appui (47), avantageusement amovibles, adaptées à coopérer avec les côtés du visage et/ou avec les oreilles de l'utilisateur.

4. Ensemble selon l'une quelconque des revendications précédentes, dans lequel ledit organe de positionnement (40) comporte une structure de support (44) formant une surface d'appui proximale (45) adaptée à recevoir, lors de l'actionnement, au moins un doigt, avantageusement deux doigts, de l'utilisateur.

5. Ensemble selon la revendication 4, dans lequel ladite structure de support (44) forme ledit au moins un manchon creux (41) et ladite seconde zone d'appui facial (46).

6. Ensemble selon la revendication 4 ou 5, dans lequel ledit organe de positionnement (40) comporte un élément de liaison (43) reliant ladite structure de support (44) à ladite première zone d'appui facial (42).

7. Ensemble selon la revendication 6, dans lequel ledit élément de liaison (43) est réglable en trois dimensions par rapport à ladite structure de support (44).

8. Ensemble selon l'une quelconque des revendications précédentes, dans lequel l'angle d'insertion vertical de la tête de distribution (20) dans la narine est compris entre 30° et 60°, avantageusement entre 40° et 50°, de préférence environ 45°.

9. Ensemble selon l'une quelconque des revendications précédentes, dans lequel l'angle d'insertion horizontal de la tête de distribution (20) dans la narine est compris entre 0° et 10°, avantageusement environ 5°.

10. Ensemble selon l'une quelconque des revendications précédentes, dans lequel la profondeur d'insertion (P) de la tête de distribution (20) dans la narine est comprise entre 5 mm et 15 mm, avantageusement environ 10 mm.

11. Ensemble selon l'une quelconque des revendications précédentes, dans lequel ledit réservoir contient une seule ou seulement deux dose(s) de produit fluide.

12. Ensemble selon l'une quelconque des revendications 1 à 10, dans lequel ledit réservoir contient une pluralité de doses de produit fluide.

## Patentansprüche

1. Abgabeanordnung aufweisend eine Vorrichtung zur nasalen Abgabe eines Fluidprodukts aufweisend einen Körper (10), der einen Behälter bildet, der Fluidprodukt enthält oder einen derartigen Behälter fest trägt, wobei ein Abgabekopf (20) an den Körper (10) angefügt ist, wobei der Abgabekopf (20) mit einer Abgabeöffnung (21) versehen ist, die Anordnung aufweisend ein Positionierungselement (40), das mit der Vorrichtung zur nasalen Abgabe zusammenwirkt, das Positionierungselement (40) aufweisend mindestens einen ersten Gesichtsauflagebereich (42), der bei der Betätigung mit der Stirn oder dem Nasenansatz des Benutzers zusammenwirkt, **dadurch gekennzeichnet, dass** der Abgabekopf (20) bezogen auf den Körper (10) axial bewegbar ist, das Positionierungselement (40) aufweisend mindestens eine hohle Hülse (41), in die der Abgabekopf (20) klemmend eingeführt ist, das Positionierungselement (40) aufweisend einen zweiten Gesichtsauflagebereich (46), der geeignet ist, mit der Oberlippe des Benutzers zusammenzuwirken.

2. Anordnung nach Anspruch 1, wobei das Positionierungselement (40) zwei hohle Hülsen (41) aufweist, die jeweils für ein jeweiliges Nasenloch angepasst sind.

3. Anordnung nach einem der vorhergehenden Ansprüche, wobei das Positionierungselement (40) dritte, vorteilhafterweise abnehmbare Auflagebereiche (47) aufweist, die geeignet sind, mit den Seiten des Gesichts und/oder den Ohren des Benutzers zusammenzuwirken.

4. Anordnung nach einem der vorhergehenden Ansprüche, wobei das Positionierungselement (40) eine Stützstruktur (44) aufweist, die eine proximale Auflagefläche (45) bildet, die geeignet ist, bei der Betätigung mindestens einen, vorteilhafterweise zwei Finger des Benutzers aufzunehmen.

5. Anordnung nach Anspruch 4, wobei die Stützstruktur (44) die mindestens eine hohle Hülse (41) und den zweiten Gesichtsauflagebereich (46) bildet.

6. Anordnung nach Anspruch 4 oder 5, wobei das Positionierungselement (40) ein Verbindungselement (43) aufweist, das die Stützstruktur (44) mit dem ersten Gesichtsauflagebereich (42) verbindet.

7. Anordnung nach Anspruch 6, wobei das Verbindungselement (43) bezogen auf die Stützstruktur (44) in drei Dimensionen einstellbar ist.

8. Anordnung nach einem der vorhergehenden Ansprüche, wobei der vertikale Einführwinkel des Abgabekopfes (20) in das Nasenloch zwischen 30° und 60°, vorteilhafterweise zwischen 40° und 50°, vorzugsweise ungefähr 45° beträgt.

9. Anordnung nach einem der vorhergehenden Ansprüche, wobei der horizontale Einführwinkel des Abgabekopfes (20) in das Nasenloch zwischen 0° und 10°, vorteilhafterweise ungefähr 5° beträgt.

10. Anordnung nach einem der vorhergehenden Ansprüche, wobei die Einführtiefe (P) des Abgabekopfes (20) in das Nasenloch zwischen 5 mm und 15 mm, vorteilhafterweise ungefähr 10 mm beträgt.

11. Anordnung nach einem der vorhergehenden Ansprüche, wobei der Behälter eine einzelne Dosis oder nur zwei Dosen Fluidprodukt enthält.

12. Anordnung nach einem der Ansprüche 1 bis 10, wobei der Behälter eine Mehrzahl von Dosen Fluidprodukt enthält.

## Claims

1. A dispenser assembly comprising a nasal fluid dispenser device having a reservoir forming body (10) containing fluid or that supports such a reservoir in stationary manner, a dispenser head (20) being assembled on said body (10), said dispenser head (20) being provided with a dispenser orifice (21), said assembly further comprising a positioner member (40) that co-operates with said nasal dispenser device, said positioner member (40) including at least a first facial bearing zone (42) that, during actuation, co-operates with the forehead or the bridge of the user's nose, the assembly being **characterized in that** said dispenser head (20) is movable axially relative to said body (10), said positioner member (40) including at least one hollow sleeve (41) in which said dispenser head (20) is inserted in clamping manner, said positioner member (40) including a second facial bearing zone (46) that is adapted to co-operate with the user's top lip.

2. An assembly according to claim 1, wherein said positioner member (40) includes two hollow sleeves (41), each adapted to a respective nostril.

3. An assembly according to any preceding claim, wherein said positioner member (40) includes third bearing zones (47), that are advantageously removable, and that are adapted to co-operate with the sides of the face and/or with the ears of the user.

4. An assembly according to any preceding claim, wherein said positioner member (40) includes a support structure (44) that forms a proximal bearing surface (45) that, during actuation, is suitable for receiving at least one, and advantageously two, of the user's fingers.

5. An assembly according to claim 4, wherein said support structure (44) forms said at least one hollow sleeve (41) and said second facial bearing zone (46).

6. An assembly according to claim 4 or claim 5, wherein said positioner member (40) includes a connection element (43) that connects said support structure (44) to said first facial bearing zone (42).

7. An assembly according to claim 6, wherein said connection element (43) is adjustable in three dimensions relative to said support structure (44).

8. An assembly according to any preceding claim, wherein the vertical insertion angle for inserting the dispenser head (20) into the nostril lies in the range 30° to 60°, advantageously in the range 40° to 50°, preferably about 45°.

9. An assembly according to any preceding claim, wherein the horizontal insertion angle for inserting the dispenser head (20) into the nostril lies in the range 0° to 10°, advantageously about 5°.

10. An assembly according to any preceding claim, wherein the insertion depth (P) for inserting the dispenser head (20) into the nostril lies in the range 5 mm to 15 mm, advantageously about 10 mm.

11. An assembly according to any preceding claim, wherein said reservoir contains only a single dose or only two doses of fluid.

12. An assembly according to any one of claims 1 to 10, wherein said reservoir contains a plurality of doses of fluid.
